# EUROPEAN PATENT APPLICATION

(11) **EP 1 006 363 A2**
(43) Date of publication of application: **07.06.2000**
(21) Application number: 99123786.8
(22) Date of filing: 30.11.1999
(51) Int. Cl.: G01N 33/543, C12Q 1/68

(54) **Biochip and method for producing the same**

(30) Priority: 01.12.1998 JP 34160498
(71) Applicant: Hitachi Software Engineering Co., Ltd., Yokohama-shi, Kanagawa 231-8475 (JP)
(72) Inventor: Ito, Toshiaki, c/o Hitachi Software Eng. Co., Ltd., Yokohama-shi, Kanagawa 231-8475 (JP); Yamamoto, Kenji, Hitachi Software Eng. Co., Ltd., Yokohama-shi, Kanagawa 231-8475 (JP); Watanabe, Toshimasa, Hitachi Software Eng. Co. Ltd, Yokohama-shi, Kanagawa 231-8475 (JP); Yurino Noriko , Hitachi Software Eng. Co. Ltd, Yokohama-shi, Kanagawa 231-8475 (JP)
(74) Representative: Liesegang, Roland, Dr.-Ing.

(57) **Abstract**

A biochip comprising probes spotted on a plate at a plurality of positions by using a binding agent for binding the probes to the plate, wherein the binding agent is locally spotted at positions where the probes are spotted.

## Description

### FIELD OF THE INVENTION

The present invention relates to a biochip comprising a plate spotted with various probes.

### BACKGROUND OF THE INVENTION

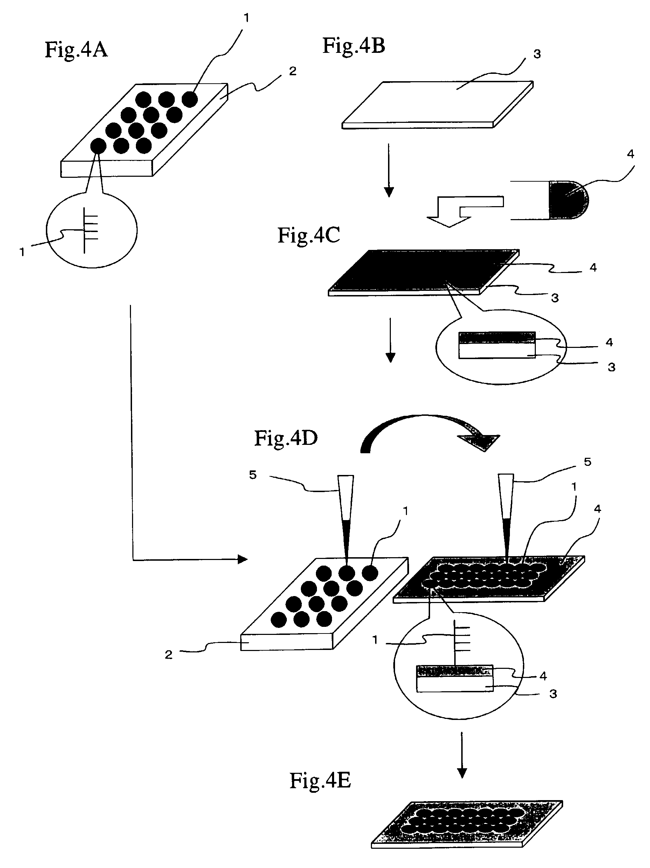

Conventionally, biochips are produced by spotting various biopolymer probes such as DNAs, RNAs and proteins on a plate (e.g., a glass plate). Figures **4A** to **4E** are diagrams for illustrating the principle of such conventional technique. First, a microplate **2** containing various probe DNAs **1** (Figure **4A**) and a glass plate **3** (Figure **4B**) are prepared. As shown in Figure **4C**, the surface of the glass plate **3** is coated with poly-1-lysine binding agent **4** for binding the DNAs **1** to the glass plate **3**. Thereafter, each of the probe DNAs **1** in the microplate **2** is transferred by a pin **5** and spotted onto the glass plate **3** coated with the poly-1-lysine binding agent **4** (Figure **4D**). This process is repeated for all of the probe DNAs **1** in the microplate **2**, thereby producing a biochip shown in Figure **4E**. In such manner, the binding agent for binding DNA to the glass plate is conventionally coated on the entire surface of the plate before spotting the DNAs on the plate.

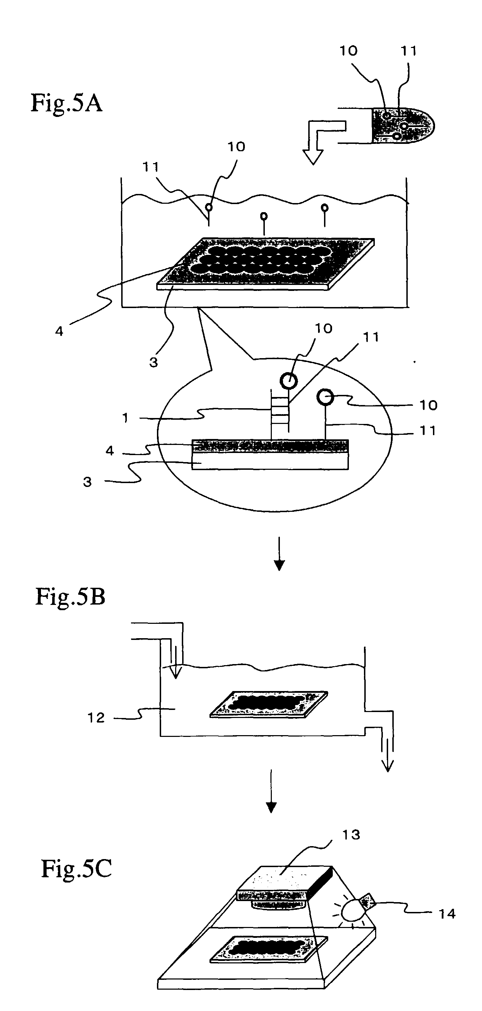

Figures **5A** to **5C** are diagrams for illustrating the principle of hybridization using the biochip. Referring to Figure **5A**, sample DNA **11** labeled with a fluorescent substance **10** is hybridized in a hybridization solution with the probe DNAs **1** that are spotted onto the glass plate **3** of the biochip via the binding agent **4**. The hybridization solution contains formaldehyde, SSC (NaCl, trisodium citrate), SDS (sodium dodecyl sulfate), EDTA (ethylenediamidete traacetic acid), distilled water and the like where the mixing ratio depends on the characteristics of the DNA used.

When the sample DNA **11** is complementary to any one of the probe DNAs **1** on the biochip, it binds to that DNA on the biochip and forms a duplex. The sample DNA **11** does not bind to probe DNAs that are not complementary thereto. However, the sample DNA **11** may bind to the binding agent **4** coating the glass plate **3**, thereby remaining as garbage.

As shown in Figure **5B**, the glass plate **3** of the biochip hybridized with the sample DNA **11** is washed in water **12** to remove the sample DNA **11** that is not bound to the probe DNAs **1**. Referring to Figure **5C**, the fluorescent substance **10** labeling the sample DNA **11** bound to the probe DNA **1** is excited with light from a lamp **14**. The fluorescent light emanated from the fluorescent substance **10** is detected by an optical sensor **13** such as a CCD to detect the presence of hybridization.

In a laboratory, the sample DNA **11** is poured onto the biochip to allow hybridization with the probe DNAs **1** spotted on the biochip followed by detection of the probe DNA bound by the sample DNA **11**. Following the hybridization and prior to the detection, the biochip is washed with water to remove the sample DNA **11** that did not bind to the probe DNAs. However, since the entire surface of the glass plate **3** is coated with the binding agent **4** for binding the probe DNA to the glass plate **3**, sample DNA **11** adheres to the binding agent area of the glass plate **3** where the probe DNAs **1** are not located. The sample DNA **11** bound to the binding agent **4** cannot be removed from the glass plate **3** by washing with water. Such remainder sample DNA **1** is detected as noise upon detection, rendering the detection sensitivity poor. In other words, some of the sample DNA **11** that is not specific to the probe DNA binds to and remains on the biochip via the binding agent **4** as garbage. When the fluorescent substance **10** labeling the sample DNA **11** bound to the binding agent **4** is excited, the fluorescent light therefrom is detected as noise, whereby S/N (signal-to-noise) ratio is lowered.

The present invention aims to solve this problem, and provides a biochip in which sample DNA does not bind to area of the plate where the probes are not located. The present invention also provides a method for producing such biochip.

### SUMMARY OF THE INVENTION

In order to accomplish the above object, the present invention provides a binding agent for binding probes on a plate only where the probes are to be spotted. Since no binding agent is provided on the portions of the plate where the probes are not to be spotted, the sample DNA that does not bind to the probe upon hybridization can be removed away from the biochip by washing with water. Therefore, noise produced upon detection can be eliminated and thus the S/N ratio can be enhanced for high sensitivity.

A biochip according to the present invention includes probes spotted on a plate at a plurality of positions by using a binding agent for binding the probes to the plate, wherein the binding agent is locally spotted at positions where the probes are spotted.

In a preferred embodiment of the inventive biochip the material of the plate is selected from the group comprising glass, nylon membranes, silicone wafer, polyimide resin and polymer plastic.

In a further preferred embodiment of the inventive biochip the binding agent is selected from the group comprising poly-1-lysine, carbodiimide and silylation-coating.

A method for producing a biochip by spotting probes on a plate by using a binding agent for binding the probes to the plate according to the present invention includes a step of spotting mixtures of respective probes and the binding agent on the plate.

An alternative method for producing a biochip by spotting probes on a plate according to the present invention includes the steps of: spotting a binding agent for binding the probes to the plate at positions where the probes are to be spotted; and spotting the probes on the plate at positions where the binding agent is spotted.

The plate used in the inventive methods may be made of a material selected from the group comprising glass, nylon membranes, silicone wafer, polyimide resin and polymer plastic.

In a preferred embodiment of the inventive methods the binding agent is selected from the group comprising poly-1-lysine, carbodiimide and silylation-coating.

The probes are preferably spotted by using the inventive pin.

The problem underlying the present invention is also solved by a pin used for spotting a probe on a plate, wherein a tip of the tip comprises at least a recess.

In one embodiment of the inventive pin the recess is of a concave shap.

In a second embodiment of the inventive pin the recess comprises at least one groove.

In a third embodiment the recess comprises a radially-shaped groove.

According to the invention, a pin used for spotting a probe on a plate has a tip provided with at least one groove. For example, the groove may be a radially-shaped groove such as a cross-shaped groove.

This specification includes all or part of the contents as disclosed in the specification and/or drawings of Japanese Patent Application No. 10-341604, which is a priority document of the present application.

### BRIEF DESCRIPTION OF THE DRAWINGS

Figures **1A** to **1E** are schematic diagrams showing the principle of one embodiment of the present invention;
Figures **2A** to **2E** are diagrams showing the principle of another embodiment of the present invention;
Figures **3A** to **3C** are diagrams for illustrating the principle of the hybridization and detection using the biochip of the invention;
Figures **4A** to **4E** are diagrams for illustrating the principle of a method for producing a conventional biochip;
Figures **5A** to **5C** are diagrams for illustrating the principle of hybridization and detection using the conventional biochip; and
Figures **6A** to **6C** are schematic diagrams showing shapes of a tip (i.e., a portion where probes are to be contacted and carried) of a pin according to the invention.

### DETAILED DESCRIPTION OF THE INVENTION

Hereinafter, the present invention will be described in more detail by way of examples with reference to the accompanying drawings. In the examples, DNA is used as a probe although the probe is not limited thereto, and RNA or protein may also be used as a probe. Although a glass plate is used in the examples, a nylon membrane or the like may also be used.

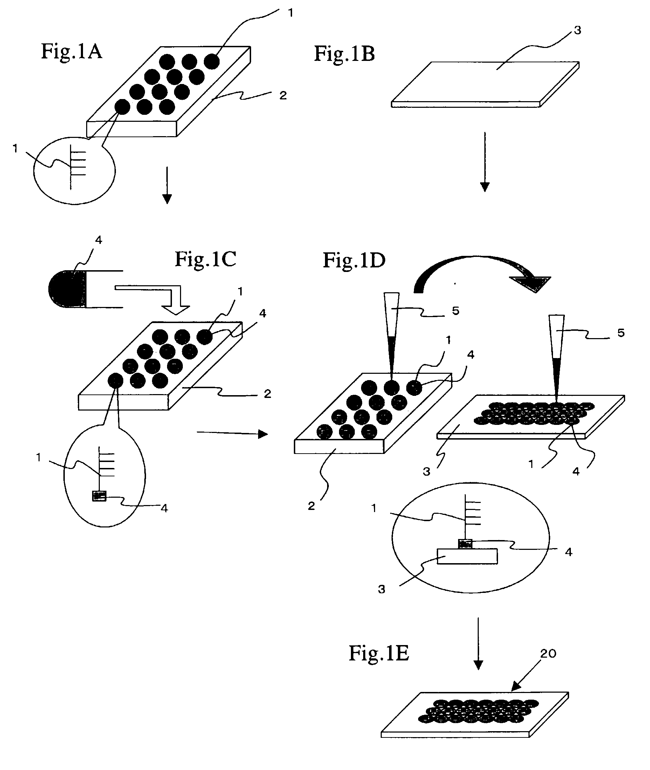

Figures **1A** to **1E** are schematic diagrams showing the principle of a first embodiment of the present invention. As shown in Figure **1A**, a microplate **2** contains various probe DNAs **1**. A plate **3** to be incorporated into the biochip shown in Figure **1B** is made of glass. Referring to Figure **1C**, a binding agent **4** for binding DNA to glass is dispensed into each well of the microplate **2** to be mixed therein with each of the probe DNAs **1**. The binding agent **4** may be, for example, poly-1-lysine or carbodiimide.

Then, as shown in Figure **1D**, each of the mixtures of the binding agent **4** and the probe DNAs **1** is suctioned by a pin **5** (or contacted and carried by the tip of the pin **5**) and spotted onto the plate **3**. This process is repeated for all of the probe DNAs **1** in the microplate **2**, thereby producing a biochip **20** shown in Figure **1E** in which the binding agent **4** is present only at the desired portions and is not present at portions where there is no probe.

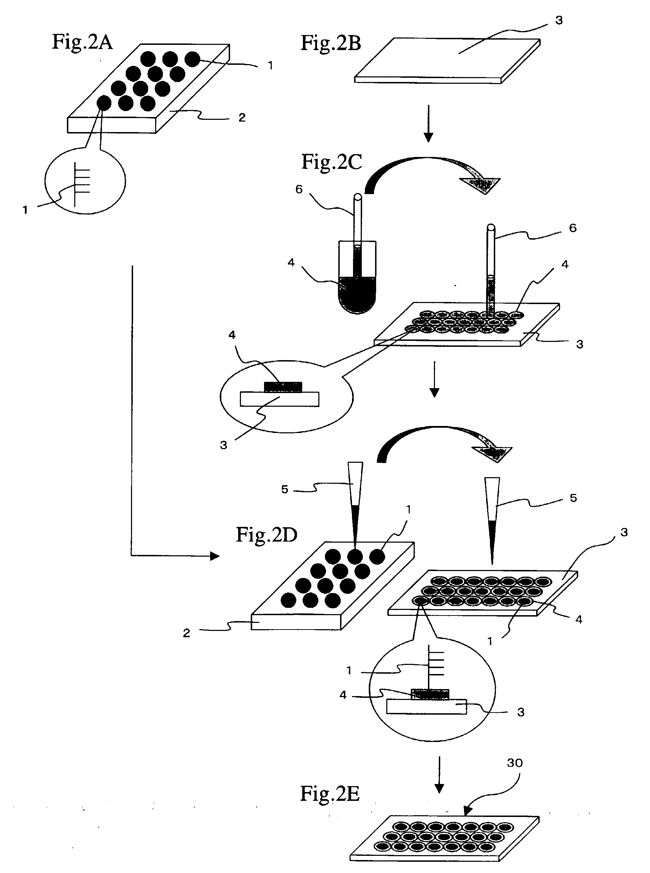

Figures **2A** to **2E** are diagrams showing the principle of a second embodiment of the present invention. A microplate **2** containing various probe DNAs **1** (Figure **2A**) and a plate **3** made of glass (Figure **2B**) are prepared. As shown in Figure **2C**, a binding agent is suctioned by, for example, a capillary tube **6** and applied on the glass plate **3** at positions where the probe DNAs are to be spotted. Then, as shown in Figure **2D**, the probe DNAs **1** in the microplate **2** are suctioned with the pin **5** (or is contacted and carried by the tip of the pin **5**) and spotted onto the plate **3**. This process is repeated for all of the probe DNAs **1** in the microplate **2**, thereby producing a biochip **30** in which the binding agent **4** is not provided on portions other than portions where the probe DNAs **1** are present (Figure **2E**).

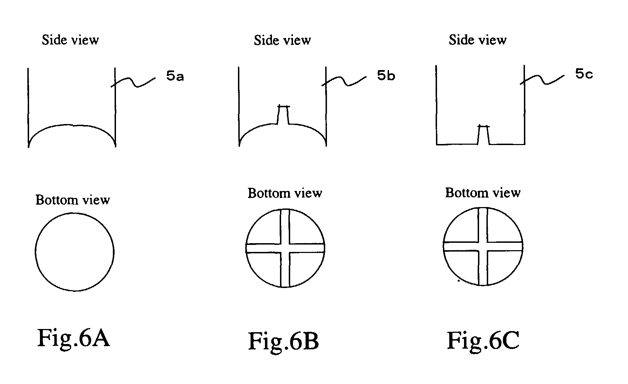

Figures **6A** to **6C** are schematic diagrams showing shapes of a tip (i.e, a portion where probes are to be contacted) of a pin **5** according to the invention. Figure **6A** shows a pin **5a** with a concave tip. A pin **5b** shown in Figure **6B** has a concave tip with a cross-shaped groove. The concave shape of the tip of the pin allows the probe solution to be carried by surface tension by simply dipping the pin in the solution. The depth of the concave is optional. The amount of the DNA carried with the pin **5a** or **5b** with the concave tip is about 10 times or more the amount carried with a conventional pin with a flat tip. A pin **5c** shown in Figure **5C** has a flat tip with a cross-shaped groove. The amount of the DNA carried with this pin **5c** is also higher than that carried with the conventional flat tip.

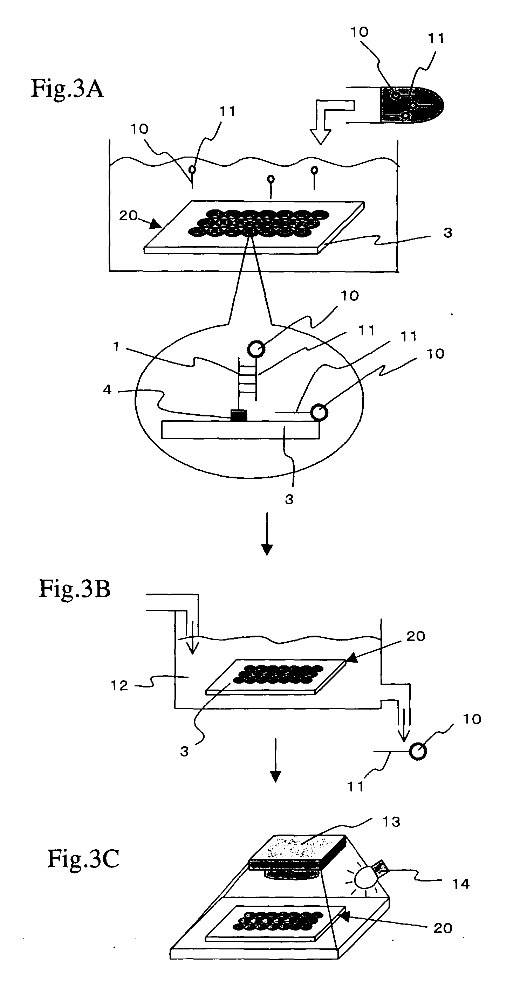

Figures **3A** to **3C** are diagrams for illustrating the principle of hybridization using the biochip **20** of the invention. Referring to Figure **3A**, a sample DNA **11** labeled with a fluorescent substance **10** is placed together with the biochip **20** in a hybridization solution for hybridization. The probe DNAs **1** are spotted on the glass plate **3** via the binding agent **4** in the biochip 20. The hybridization solution contains formaldehide, SSC (NaCl, trisodium citrate), SDS (sodium dodecyl sulfate), EDTA (ethylenediamidetetraacetic acid) and distilled water where the mixing ratio differs depending on the characteristic of the DNA used.

When the sample DNA **11** and any one of the probe DNAs **1** on the biochip **20** are complementary to each other, both DNAs bind to each other and form a duplex. On the other hand, when the sample DNA **11** and any one of the probe DNAs **1** are not complementary to each other, the sample DNA **11** does not bind to that probe DNA **1** and remain as garbage. As shown in Figure **3B**, the sample DNA **11** labeled with the fluorescent substance **10** remaining on the glass plate **3** is washed away in water **12**. Since the binding between the glass and the DNA is weak, the remaining garbage sample **11** that is not bound to the probe DNAs **1** is removed away. Referring to Figure **3C**, the fluorescent substance **10** labeling the sample DNA **11** bound to the probe DNA **1** is excited with light from a lamp **14**. The fluorescent light emanated from the fluorescent substance **10** is detected by an optical sensor **13** such as a CCD to detect the presence of hybridization. Since there is no garbage sample DNA left on the biochip **20**, the S/N ratio upon detection is enhanced.

According to the present invention, a biochip can be produced in which a binding agent is locally spotted only where probes are to be spotted. Thus, the detection sensitivity upon reading the biochip can be enhanced.

All publications, including patent and patent application cited herein are incorporated herein by reference in their entirety.

The features disclosed in the foregoing description, in the claims and/or in the accompanying drawings may, both separately and in any combination thereof, be material for realising the invention in divers forms thereof.

## Claims

1. A biochip comprising probes spotted on a plate at a plurality of positions by using a binding agent for binding the probes to the plate, wherein the binding agent is locally spotted at positions where the probes are spotted.

2. The biochip according to claim 1, wherein the material of the plate is selected from the group comprising glass, nylon membranes, silicone wafer, polyimide resin and polymer plastic.

3. The biochip according to claim 1 or 2, wherein the binding agent is selected from the group comprising poly-1-lysine, carbodiimide and silylation-coating.

4. A method for producing a biochip by spotting probes on a plate by using a binding agent for binding the probes to the plate, the method comprising a step of spotting mixtures of respective probes and the binding agent on the plate.

5. A method for producing a biochip by spotting probes on a plate, the method comprising the steps of:
spotting a binding agent for binding the probes to the plate at positions where the probes are to be spotted; and
spotting the probes on the plate at positions where the binding agent is spotted.

6. The method for producing a biochip according to claim 4 or 5, wherein the plate is made of a material which is selected from the group comprising nylon membranes, glass, silicone wafer, polyimide resin and polymer plastic.

7. The method according to any of claims 4 to 6, wherein the binding agent is selected form the group comprising poly-1-lysine carbodiimide and silylation-coating.

8. The method for producing a biochip according to any one of claims 4 to 7, wherein the probes are spotted by using a pin with a recessed tip.

9. A pin used for spotting a probe on a plate, wherein a tip of the pin comprises at least one recess.

10. The pin according to claim 9, wherein the recess is of a concave shape.

11. The pin according to claim 9, wherein the recess comprises at least one groove.

12. The pin according to claim 9 or 11, wherein the recess comprises a radially-shaped groove.
